# EUROPEAN PATENT APPLICATION

(11) **EP 1 582 203 A1**
(43) Date of publication of application: **05.10.2005**
(21) Application number: 05252065.7
(22) Date of filing: 01.04.2005
(51) Int. Cl.: A61K 7/48

(54) **Cosmetic compositions containing swelled silicone elastomer powders and gelled block copolymers**

(30) Priority: 01.04.2004 US 816574
(71) Applicant: L'OREAL S.A., 75008 Paris Cedex (FR)
(72) Inventor: Lu, Shao Xiang, Plainsboro New Jersey 08536 (US)
(74) Representative: Bentham, Stephen

(57) **Abstract**

Disclosed are compositions comprising a gelled block copolymer and a silicone elastomer powder, and methods of making and using them.

## Description

### BACKGROUND OF THE INVENTION

Many cosmetic compositions, including pigmented cosmetics such as foundations, concealers, lipsticks, mascaras, and other cosmetic and sunscreen compositions, have been developed for comfortable application and wear. However many of these compositions are difficult to apply and do not have a smooth feel upon application. Furthermore, compositions may have a tendency to be tacky, resulting in poor application and spreadability characteristics, and fail to provide acceptable wear resistance. U.S. Patent 6,083,516 teaches wear resistant cosmetics containing a styrene-ethylene/propylene mixed block copolymer, mixed with isododecane, and which can be formulated with a cosmetic carrier such as a silicone base such as dimethicone or cyclomethicone or an organic volatile in an aqueous base such as isoparaffin.

### SUMMARY OF THE INVENTION

One aspect of the present invention is directed to a cosmetic composition, comprising a gelled block copolymer having at least one hard segment and at least one soft segment; and a silicone elastomer powder comprising a silicone elastomer core having coated thereon a silicone resin, wherein said powder is swelled with a swelling agent.

Another aspect of the present invention is directed to a method for care, make-up or treatment of a keratin material, comprising applying to the keratin material a composition comprising a gelled block copolymer having at least one hard segment and at least one soft segment; and a silicone elastomer powder comprising a silicone elastomer core coated with a silicone resin, wherein said powder is swelled with a swelling agent.

The compositions of the present invention may take a variety of forms of purposes of finished products. For example, the compositions may take any number of forms, including a paste, a gel (e.g., a solid, rigid or supple gel, including an anhydrous gel such as a translucent anhydrous gel or a transparent anhydrous gel), a cream, an emulsion (an aqueous or anhydrous emulsion), a solid (e.g., a molded composition or cast as a stick (e.g., a poured or molded stick), a compact, a dish, or a powder (e.g., a loose, compact or pressed powder). In addition, while compositions of the invention are described in terms of being cosmetic compositions, to the extent that they are intended to be applied to skin, they may also be considered as dermatological compositions, particularly if they contain a drug or other active agent considered to treat or benefit skin.

Applicants have discovered that some solvents that cause block copolymers to gel, such as oils or other emollients, may not be compatible with other cosmetically acceptable solvents such as dimethicone, especially high molecular weight dimethicones. Applicants have also discovered that these ingredients are compatible when the swelling agent is incorporated into the compositions in conjunction with the silicone elastomer powder. The results are that embodiments of the present invention have relatively little phase separation, more aesthetic appeal and better application and spreadability characteristics.

### DETAILED DESCRIPTION

Gelled block copolymers useful in the present invention include di-block, tri-block, multi-block, radial and star block copolymers, and mixtures and blends thereof. The copolymers have at least two thermodynamically incompatible segments. These segments are also known in the art as "thermoplastic" or "hard" segments, and "elastomeric" or "soft" segments. Aside from their compositional nature, they differ in terms of having relatively high glass transition temperatures, Tg, (e.g., typically at least about 60 °C), and relatively low glass transition temperatures (e.g., typically no higher than about room temperature, e.g., 25 °C). See, e.g., U.S. Patents 5,294,438 and 6,403,070. Examples of hard segments include polystyrene, polymethacrylate and polyacrylate. Examples of soft segments include ethylene/propylene copolymers, ethylene/butylene copolymers, propylene/butylene copolymers, polybutadiene, polyisoprene, polymers of hydrogenated butadienes and isoprenes, and mixtures thereof. A di-block is usually defined as A-B type or a hard segment (A) followed by a soft segment (B) in sequence. A tri-block is usually defined as an A-B-A type copolymer or a ratio of one hard, one soft, and one hard segment. Multi-block or radial or star copolymer film formers usually contain any combination of hard and soft segments, provided that there are both hard and soft characteristics.

In some embodiments, the block copolymer of the present invention is chosen from the class of Kraton™ rubbers (Shell Chemical Company) or from similar gelling agents. Kraton™ rubbers are thermoplastic elastomers in which the polymer chains comprise a tri-block, di-block, or radial or star block configuration or numerous mixtures thereof. The Kraton™ tri-block rubbers have polystyrene segments on each end of a rubber segment, while the Kraton™ di-block rubbers have a polystyrene segment attached to a rubber segment. The Kraton™ radial or star configuration may be a four-point or other multipoint star made of rubber with a polystyrene segment attached to each end of a rubber segment. The configuration of each of the Kraton™ rubbers form separate polystyrene and rubber domains.

Each molecule of Kraton™ rubber is said to comprise block segments of styrene monomer units and rubber monomer and/or co-monomer units. The most common structure for the Kraton™ triblock copolymer is the linear A-B-A block type styrene-butadiene-styrene, styrene-isoprene-styrene, styrene-ethylenepropylene-styrene, or styrene-ethylenebutylene-styrene. The Kraton™ di-block is preferably the AB block type such as styrene-ethylenepropylene, styrene-ethylenebutylene, styrene-butadiene, or styrene-isoprene. The Kraton™ rubber configuration is well known in the art and any block copolymer film former with a similar configuration is within the practice of the invention. Other block copolymers are sold under the tradename Septon (which represent elastomers known as SEEPS, sold by Kurary, Co., Ltd.

Other gelled block copolymers useful in the present invention preferred embodiments include the use of block copolymer film formers comprising a styrene/butylene/ethylene/styrene copolymer (tri-block), an ethylene/propylene/styrene copolymer (radial or star block) or a mixture or blend of the two. (Some manufacturers refer to block copolymers as hydrogenated block copolymers, e.g. hydrogenated styrene/butadiene/styrene copolymer and hydrogenated styrene/isoprene/styrene copolymer (tri-block). Specific examples include Versagel M5960 and Versagel M5970, commercially available from Penreco of Houston Texas. The block copolymers are available from Brooks Industries, such as Gel Base (e.g., Code 05895, which is a styrene-ethylene/propylene mixed block copolymer already in combination with a solvent, namely isododecane) .

By the term "gelled", it is meant that the block copolymer is dissolved in a solvent. The block copolymer is formulated by dissolving it in a solvent such as oils, hydrocarbon solvents and esters. Hydrocarbons useful in the practice of the invention include but are not limited to mineral oils, mineral solvents, mineral spirits, petroleum, waxes, synthetic hydrocarbons, animal oils, vegetable oils, and mixtures thereof. In some embodiments, the block copolymer is formulated by dissolving the block copolymer in isododecane or a light paraffinic solvent. In other embodiments, the block copolymer film former may be formulated by dissolving the block copolymer in a non-hydrocarbon solvent such as amyl acetate, butyl acetate, isobutyl acetate, ethyl acetate, propyl acetate or isopropyl acetate. The solvent and solubility conditions for formulating a block copolymer film former from a block copolymer will be chosen by a person skilled in the art in order to prepare a composition which has the desired properties. One of ordinary skill in the art will be able to determine the solubility parameters and choose a solvent based on the block copolymer chosen for the envisaged application. More information regarding solubility parameters and solvents useful in the processing of specific block copolymers is available from the various manufacturers of block copolymers, e.g., Shell Chemical Company. Additional discussions of polymer solubility parameter concepts are presented in: *Encyclopedia of Polymer Science and Technology,* Vol. 3, Interscience, New York (1965) and *Encyclopedia of Chemical Technology,* Supp. Vol., Interscience, New York (1971).

Generally, one or more gelled block copolymers (i.e., the block copolymer(s) and the solvent(s)) are present in the compositions of the present invention in amounts from about 0.1% to about 95% or more, and preferably from about 0.5% to about 70% by weight of the composition. The amounts of the block copolymer or copolymers, as well as their structure (di-block, tri-block, etc.), affect the nature of the gel, which may range from a fragile or a soft gel through flexible gels, and to firm gels. For instance, soft gels contain relatively high amounts of soft segments, and firm gels contain relatively high amounts of hard segments. Generally, the amount of hard segments ranges from about 10 % to about 40 %, and more preferably from about 15 to about 30 % by weight of the total weight of the block copolymer(s). The overall properties of the composition may also be affected by including more than one such block copolymer e.g., including a mixture of copolymers. For example, presence of tri-block copolymers enhances integrity of the film formed. The gel may also be transparent, translucent or opaque, depending upon the other cosmetically acceptable ingredients added, as described herein. Persons skilled in the art will be determine amounts of the block co-polymer and choose additional ingredients based on the final form of the cosmetic product. See, e.g., U.S. Patents 5,959,009 (eyelash compositions); 5,756,082 (sticks); 6,060,072 (transfer-resistant, color compositions); 5,578,299 (rinse-off skin conditioner); 6,403,070 (anhydrous deodorant compositions); 6,423,306 (transfer-free cosmetics); and 5,294,438 (lubricating and moisturizing shaving preparations).

The silicone elastomer powders useful in this invention comprise particles of a globular or spherical core of cured silicone elastomer particle that, in general, have an average particle diameter from 0.1 *µ*m to 100 *µ*m, wherein the core is coated with a silicone resin e.g., a coating layer formed of a polyorganosilsesquioxane resin, which in general, is present in an amount of from 1 to 500 parts by weight per 100 parts by weight of the core silicone elastomer.

In certain embodiments, the silicone elastomer forming the core particles is a cured diorganopolysiloxane having linear diorganopolysiloxane segments represented by the general formula (I):

(R-Si-O)ₐ (I);

wherein each R is, independently from the others, an unsubstituted or substituted monovalent hydrocarbon group having 1 to 20 carbon atoms exemplified by alkyl groups such as methyl, ethyl, propyl and butyl groups, aryl groups such as phenyl and tolyl groups, alkenyl groups such as vinyl and allyl groups and aralkyl groups such as 2-phenylethyl and 2-phenylpropyl groups as well as those substituted hydrocarbon groups obtained by replacing a part or all of the hydrogen atoms in the above named hydrocarbon groups with substituents including halogen atoms, epoxy group, amino group, mercapto group, (meth)acryloxy group and the like such as chloromethyl and 3,3,3-trifluoropropyl groups, at least 90% by moles of the groups R being preferably methyl groups, and the subscript a is a positive integer in the range of from 5 to 5000 such as from 10 to 1000.

The coated silicone elastomer particles can be prepared by in situ hydrolysis and condensation reaction of a trialkoxy silane compound in the presence of the cured silicone elastomer particles in an aqueous dispersion so as to form the coating layer of a silicone e.g., polyorganosilsesquioxane, resin on the surface of the silicone elastomer particles. The method of preparation includes admixing an aqueous dispersion of particles of a cured silicone elastomer having an average particle diameter in the range from 0.1 *µ*m to 100 *µ*m with an alkaline compound and a trialkoxy silane compound represented by the general formula (II) :

R'-Si(OR")₃ (II);

wherein R' is an unsubstituted or substituted monovalent hydrocarbon group, and R" is an alkyl group having 1 to 6 carbon atoms, at a temperature not exceeding 60 °C, and under agitation. Specific examples of the preparation of such silicone elastomer coated particles are described in U.S. Patent 5,538,793. Examples of commercially available silicone elastomer particles coated with polyorganosilsesquioxane are available from Shin-Etsu and include the KSP-100 series, KSP-200 series and KSP-300 series. These are spherical particles of silicone elastomer coated with silicone resin, wherein the silicone elastomer core can be unfunctionalized for the KSP-100 series, functionalized with fluoroalkyl groups for the KSP-200 and functionalized with phenyl groups in the case of the KSP-300. The ratio of the amount of the silicone elastomer powder to the one or more gelled block copolymers generally ranges from about 0.1 to about 9.0. In some embodiments the ratio is from about 0.5 to about 5.0. In other embodiments, the ratio is from about 1.0 to about 4.0, and in yet other embodiments, the ratio is from about 1.0 to about 3.0.

Swelling agents useful in the present invention include silicone oils chosen from volatile and non-volatile, linear and cyclic polydimethylsiloxanes (PDMSs) that are liquid at room temperature (e.g., cyclomethicones and dimethicones); polydimethylsiloxanes comprising alkyl or alkoxy groups which are pendant and/or at the end of the silicone chain, the groups each containing from 2 to 24 carbon atoms; phenylsilicones such as phenyl trimethicones, phenyl dimethicones, phenyl trimethylsiloxy diphenylsiloxanes, diphenyl dimethicones, diphenyl methyldiphenyl trisiloxanes and 2-phenylethyl trimethylsiloxysilicates, and fluorinated silicones. Some low-viscosity silicone oils useful in the present invention are linear polysiloxanes consisting (except for the terminal groups) of units of formula (III): [(R)₂-Si-O] in which each of the two substituents denoted "R" independently represents a lower alkyl group (having 1 to 6 C). The degree of polymerization (number of repeating units) of these low-viscosity polysiloxanes may range for example from about 3 to 2000. These low-viscosity silicone oils can be prepared according to known methods, or bought commercially: for example series 47 Silbione oil (Rhone Poulenc), series 200 oil (Dow Corning), SF 96 oil (General Electric). The terminal groups are, for example, trimethylsilyl, dimethyl hydroxymethylsilyl or vinyl dimethylsilyl groups.

The swelling agent must be cosmetically acceptable. Aside from that criterion, the choice of swelling agent depends on the chemical nature of the silicone elastomer core. For example, silicone elastomer cores having phenyl substituents (e.g., KSP-300) may be used with swelling agents such as a phenyltrimethicone, and cores having fluoro groups (e.g., KSP-200) may be used with agents such as fluorinated silicones. On the other hand, non-functionalized silicone elastomer cores (e.g., KSP-100) may be used with non-functionalized or functionalized swelling agents. Viscosity of the swelling agent generally varies from about 5 to 100,000 cst (centistokes). Agents having a relatively high viscosity will cause relatively slow swelling of the silicone elastomer powder, and agents having low viscosity will generally cause relatively fast swelling of the elastomer powder. More than one swelling agent may be used. In general, the swelling agent is present in the cosmetic composition in an amount of from about 0.1 to about 90%, and preferably from about 0.1 to about 40% by total weight of the composition. Relative amounts of silicone elastomer powder and swelling agent are determined based on the nature of the cosmetic composition. In general, the swelling agent will cause swelling of the elastomer powder in a range from about 10% of the original volume of the powder, to about 2.5 times or more the original volume (as measured, for example, by visual observation of a phase separation of unabsorbed swelling agent and the silicone elastomer core coated with the resin, in its swollen state). Stated differently, the silicone powder will typically absorb up to about 2.5 times its own weight of the swelling agent. In general, the combined weight of silicone elastomer powder and the swelling agent ranges from about 0.1% to about 99% based on the total weight of the composition.

Separate and apart from the solvent contained in the gelled block co-polymer, the compositions of the present invention may contain at least one liquid fatty phase, which in some embodiments, may comprise at least one oil. The at least one oil, for example, may be chosen from polar oils and apolar oils including hydrocarbon-based liquid oils and oily liquids at room temperature.

For a liquid fatty phase structured with an apolar polymer of the hydrocarbon-based type, this fatty phase may contain more than 30%, for example more than 40% by weight, or from 50% to 100% by weight, of at least one liquid apolar, such as hydrocarbon-based, oil, relative to the total weight of the liquid fatty phase.

For example, the at least one polar oil useful in the invention may be chosen from:

-hydrocarbon-based plant oils with a high content of triglycerides comprising fatty acid esters of glycerol in which the fatty acids may have varied chain lengths from C₄ to C₂₄, these chains possibly being chosen from linear and branched, and saturated and unsaturated chains; these oils are chosen from, for example, wheat germ oil, corn oil, sunflower oil, karite butter, castor oil, sweet almond oil, macadamia oil, apricot oil, soybean oil, cotton oil, alfalfa oil, poppy oil, pumpkin oil, sesame oil, marrow oil, rapeseed oil, avocado oil, hazelnut oil, grape seed oil, blackcurrant seed oil, evening primrose oil, millet oil, barley oil, quinoa oil, olive oil, rye oil, safflower oil, candlenut oil, passion flower oil and musk rose oil; or alternatively caprylic/capric acid triglycerides such as those sold by Stearineries Dubois or those sold under the names Miglyol 810, 812 and 818 by Dynamit Nobel;

-synthetic oils or esters of formula R₅COOR₆ in which R₅ is chosen from linear and branched fatty acid residues containing from 1 to 40 carbon atoms and R₆ is chosen from, for example, a hydrocarbon-based chain containing from 1 to 40 carbon atoms, on condition that R₅ + R₆ ≥ 10, such as, for example, purcellin oil (cetostearyl octanoate), isononyl isononanoate, C₁₂-C₁₅ alkyl benzoates, isopropyl myristate, 2-ethylhexyl palmitate, isostearyl isostearate and alkyl or polyalkyl octanoates, decanoates or ricinoleates; hydroxylated esters such as isostearyl lactate and diisostearyl malate; and pentaerythritol esters;

-synthetic ethers containing from 10 to 40 carbon atoms;

-C₈ to C₂₆ fatty alcohols such as oleyl alcohol; and

-C₈ to C₂₆ fatty acids such as oleic acid, linolenic acid or linoleic acid.

The at least one apolar oil according to the invention may include a hydrocarbon chosen from linear and branched, volatile and non-volatile hydrocarbons of synthetic and mineral origin, such as volatile liquid paraffins (such as isoparaffins and isododecane) or non-volatile liquid paraffins and derivatives thereof, liquid petrolatum, liquid lanolin, polydecenes, hydrogenated polyisobutene such as Parleam® , and squalane; silicone oils, polydimethylsiloxanes and phenylsilicones that would otherwise not function herein as a swelling agent; and mixtures thereof. In one embodiment, apolar oils, such as an oil or a mixture of hydrocarbon oils, are chosen from those of mineral and synthetic origin, hydrocarbons such as alkanes such as Parleam® oil, isoparaffins including isododecane, and squalane, and mixtures thereof. These oils may, in one embodiment, be combined with at least one phenylsilicone oil.

The liquid fatty phase, in one embodiment, contains at least one non-volatile oil chosen from, for example, hydrocarbon-based oils of mineral, plant and synthetic origin, synthetic esters or ethers, silicone oils and mixtures thereof.

In practice, the total liquid fatty phase may be present, for example, in an amount ranging from about 0.1% to about 99% by weight relative to the total weight of the composition; further examples include ranges of from about 5.0% to about 95.5%, from about 10% to about 80%, from about 20% to about 75%, and from about 1.0% to about 60% by weight relative to the total weight of the composition.

In addition to the liquid fatty phase, the cosmetic composition may also contain an aqueous phase, in which case, the cosmetic composition will be in the form of an emulsion. In these embodiments, the composition will also contain one or more emulsifiers to facilitate formation and stability of an emulsion. Examples of aqueous emulsions include oil-in-water emulsions, water-in-oil emulsions and multiple emulsions such as oil-in-water-in-oil emulsions and water-in-oil-in-water emulsions. However, the compositions of the present invention are not limited to emulsions that contain an aqueous phase. Compositions may also be in the form of an anhydrous emulsion, (e.g., they may contain a polyol such as glycerin and propylene glycol).

Examples of organic emulsifiers include any ethoxylated surfactants known in the art such as Polysorbate-20, Laureth-7, Laureth-4, Sepigel® 305 available from SEPPIC and other similar ingredients disclosed in the International *Cosmetic Ingredient Dictionary and Handbook Vol. 4* (9^{th} ed. 2002), more particularly the emulsifiers disclosed on pages 2962-2971. Examples of organosilicone emulsifiers include cetyl dimethicone copolyol-polyglyceryl-4-isostearate-hexylaurate (ABIL® WE 09) available from Goldschmidt Chemical Corporation, Cetyl Dimethicone Copolyol (ABIL® EM 90), (ABIL® EM 97), Laurylmethicone Copolyol (5200), Cyclomethicone (and) Dimethicone Copolyol (DC 5225 C and DC 3225 C) available from GE Silicones, Cyclopentasiloxane & Dimethicone Copolyol (GE SF 1528) or any other formulation aids known to persons skilled in the art. Other fatty substances useful as formulation aids include but are not limited to, silicones in esterified or unesterified liquid form or in esterified solid form, such as behenate dimethicone; and non-silicone fatty substances including oils such as vegetable and mineral oil; animal and/or synthetic waxes such as beeswax, paraffin, rice bran wax, candelilla wax, carnauba wax and derivatives thereof; and hydrocarbon gels or bentone type gels, such as Gel SS71, Gel EA2786, Quaternium-18 Bentonite, 38 CE, Gel ISD V or Gel ISD. Other emulsifiers may include sugar derivatives such as alkylpolyglucosides or sugar esters. Also used as emulsifiers include ethoxylated stearates such as polyglyceryl-2 dipolyhydroxystearate, or polyglyceryl-10 polyhydroxystearate or PEG-30 dipolyhydroxystearate. These substances may be included in the compositions of the present invention to affect properties such as consistency and texture.

Additional film-forming polymers may be present in the composition, provided that they are compatible with it. Appropriate amounts of the film former may be determined by one of skill in the art and can vary considerably based on the application. For example, for cosmetic compositions, the film former may be used in an amount from 0.1% to 20% such as, for example, from 1% to 10% by weight, relative to the total weight of the composition.

In one embodiment, the film-forming silicone resin is chosen from silsesquioxanes and siloxysilicates. Representative examples of such silsesquioxane film formers may include Belsil PMS MK, also referred to as Resin MK, available from Wacker Chemie, KR-220L, KR-242A, or KR-251 available from SHIN-ETSU. Examples of siloxysilicate film formers may include Wacker 803 and 804 available from Wacker Silicones Corporation, G.E. 1170-002 available from General Electric, diisostearoyl trimethylolpropane siloxysilicates, such as SF 1318, available from GE Silicones. High viscosity phenylated silicone such as phenyltrimethicone available as Belsil PDM 1000 may be used as a silicone based film former.

In another embodiment the film former may be a compound obtained by the reaction of silicone moieties with ethylenically unsaturated monomers. The resulting copolymers may be graft or block copolymers comprising at least one backbone and at least one chain, wherein at least one of the at least one backbone and at least one chain is chosen from silicones. In an embodiment, the at least one copolymer is chosen from copolymers comprising at least one polar backbone and at least one non-polar chain and copolymers comprising at least one non-polar backbone and at least one polar chain, wherein at least one of the at least one backbone and at least one chain is chosen from silicones.

In an embodiment, the at least one copolymer is chosen from copolymers comprising a polymer skeleton comprising at least one non-polar, silicone backbone substituted with at least one polar, non-silicone chain and copolymers comprising a polymer skeleton comprising at least one polar, non-silicone backbone substituted with at least one non-polar, silicone chain.

In another embodiment, the at least one copolymer is chosen from copolymers comprising a polymer skeleton comprising at least one polar, silicone backbone substituted with at least one non-polar, non-silicone chain and copolymers comprising a polymer skeleton comprising at least one non-polar, non-silicone backbone substituted with at least one polar, silicone chain.

In an embodiment, the at least one polar chain comprises at least one ester group. In another embodiment, the at least one polar chain comprises at least one ester group and at least one double bond. In another embodiment, the at least one polar, non-silicone backbone is chosen from acrylate polymers, methacrylate polymers, and vinyl polymers.

In another embodiment, the at least one copolymer further comprises at least one hydrocarbon group. In an embodiment, the at least one hydrocarbon group is a terminal hydrocarbon group bonded to the polymer skeleton. In another embodiment, the at least one hydrocarbon group is a pendant hydrocarbon group bonded to the polymer skeleton. In another embodiment, the at least one hydrocarbon group is a terminal hydrocarbon group bonded to at least one chain on the polymer skeleton. In another embodiment, the hydrocarbon group is a pendant hydrocarbon group bonded to at least one chain on the polymer skeleton. Non-limiting examples of the at least one hydrocarbon group include C₅-C₂₅ alkyl groups, optionally substituted, such as C₁₈ alkyl groups and C₂₂ alkyl groups.

Non-limiting examples of the at least one copolymer include silicone/(meth)acrylate copolymers, such as those as described in U.S. Patents 5,061,481, 5,219,560, and 5,262,087. Further non-limiting examples of the at least one copolymer are non-polar silicone copolymers comprising repeating units of at least one polar (meth)acrylate unit and vinyl copolymers grafted with at least one non-polar silicone chain. Non-limiting examples of such copolymers are acrylates/stearyl acrylate/dimethicone acrylates copolymers, such as those commercially available from Shin-Etsu, for example, the product sold under the tradename KP-561, and acrylates/behenyl acrylate/dimethicone acrylates copolymer, such as those commercially available from Shin-Etsu, for example, the product sold under the tradename KP-562.

Another non-limiting example of at least one copolymer suitable for use in the present invention are silicone esters comprising units of formulae (IV) and (V), disclosed in U.S. Patents 6,045,782, 5,334,737, and 4,725,658:

Rₐ R^{E} _{b} SiO_{[4-(a+b)/2]} (IV);

and

R'ₓ R^{E} _{y} SiO_{1/2} (V);

wherein

R and R', which may be identical or different, are each chosen from optionally substituted hydrocarbon groups;

a and b, which may be identical or different, are each a number ranging from 0 to 3, with the proviso that the sum of a and b is a number ranging from 1 to 3,

x and y, which may be identical or different, are each a number ranging from 0 to 3, with the proviso that the sum of x and y is a number ranging from 1 to 3;

R^{E}, which may be identical or different, are each chosen from groups comprising at least one carboxylic ester.

In an embodiment, R^{E} groups are chosen from groups comprising at least one ester group formed from the reaction of at least one acid and at least one alcohol. In an embodiment, the at least one acid comprises at least two carbon atoms. In another embodiment, the at least one alcohol comprises at least ten carbon atoms. Non-limiting examples of the at least one acid include branched acids such as isostearic acid, and linear acids such as behenic acid. Non-limiting examples of the at least one alcohol include monohydric alcohols and polyhydric alcohols, such as n-propanol and branched etheralkanols such as (3,3,3-trimethylolpropoxy)propane.

Further non-limiting examples of the at least one copolymer include liquid siloxy silicates and silicone esters such as those disclosed in U.S. Patent 5,334,737, such as diisostearoyl trimethylolpropane siloxysilicate and dilauroyl trimethylolpropane siloxy silicate, which are commercially available from General Electric under the tradenames SF 1318 and SF 1312, respectively.

Further non-limiting examples of the at least one copolymer include polymers comprising a backbone chosen from vinyl polymers, methacrylic polymers, and acrylic polymers and at least one chain chosen from pendant siloxane groups and pendant fluorochemical groups. Non-limiting examples of such polymers comprise at least one unit derived from at least one A monomer, at least one unit derived from at least one C monomer, at least one unit derived from D monomers, and, optionally, at least one unit derived from at least one B monomer, wherein:

A, which may be identical or different, are each chosen from free-radically-polymerizable acrylic esters of at least one alcohol chosen from 1,1-dihydroperfluoroalkanols, omega-hydridofluoroalkanols, fluoroalkylsulfonamido alcohols, cyclic fluoroalkyl alcohols, and fluoroether alcohols, and analogs of any of the foregoing at least one alcohols, and free-radically-polymerizable methacrylic esters of at least one alcohol chosen from 1,1,-dihydroperfluoroalkanols, omega-hydridofluoroalkanols, fluoroalkylsulfonamido alcohols, cyclic fluoroalkyl alcohols, and fluoroether alcohols, and analogs of any of the foregoing at least one alcohols;

B, which may be identical or different, are each chosen from reinforcing monomers which are copolymerizable with at least one A monomer;

C, which may be identical or different, are each chosen from monomers represented by formula (VI):

X (Y) ₙ Si (R) ₃₋ₘ Zₘ (VI)

wherein

X is chosen from vinyl groups which are copolymerizable with at least one A monomer and at least one B monomer,

Y is chosen from divalent allylene groups, divalent arylene groups, divalent alkarylene groups, and divalent aralkylene groups, wherein the groups comprise from 1 to 30 carbon atoms, and further wherein the groups optionally further comprise at least one group chosen from ester groups, amide groups, urethane groups, and urea groups;

n is zero or 1;

m is a number ranging from 1 to 3;

R, which may be identical or different, are each chosen from hydrogen, C₁- C₄ alkyl groups, aryl groups, and alkoxy groups; and

Z, which may be identical or different, are each chosen from monovalent siloxane polymeric groups; and

D, which may be identical or different, are each chosen from free-radically-polymerizable acrylate copolymers and free-radically-polymerizable methacrylate copolymers. Such polymers and their manufacture are disclosed in U.S. Patents 5,209,924 and 4,972,037, and WO 01/32737.

Further non-limiting examples of the at least one copolymer include polymers comprising at least one A monomer, at least one C monomer, and at least one D monomer, wherein A, which may be identical or different, are each chosen from polymerizable acrylic esters of at least one fluoroalkylsulfonamido alcohol and polymerizable methacrylic esters of at least one fluoroalkylsulfonamido alcohol, D, which may be identical or different, are each chosen from methacrylic acid esters of at least one C₁-C₁₂ linear alcohol and methacrylic acid esters of at least one C₁-C₁₂ branched alcohol, and C is as defined above in preceding paragraphs. Such polymers include polymers comprising at least one group represented by formula (VII): 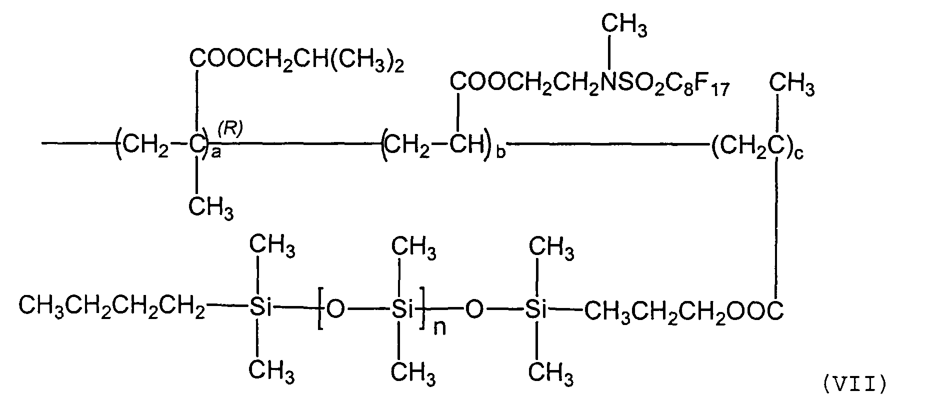 wherein

a, b, and c, which may be identical or different, are each a number ranging from 1 to 100,000; and

the terminal groups, which may be identical or different, are each chosen from C₁-C₂₀ linear alkyl groups, C₃-C₂₀ branched chain alkyl groups, C₃-C₂₀ aryl groups, C₃-C₂₀ linear alkoxy groups, and C₃-C₂₀ branched alkoxy groups. Such polymers are disclosed in U.S. Patents 4,972,037, 5,061,481, 5,209,924, 5,849,275, and 6,033,650. These polymers may be purchased from Minnesota Mining and Manufacturing Company under the tradenames "Silicone Plus" polymers. For example, poly(isobutyl methacrylate-co-methyl FOSEA)-g-poly(dimethylsiloxane) is sold under the tradename SA 70-5 IBMMF.

Other non-limiting examples of the at least one copolymer is silicone/acrylate graft terpolymers, for example, those represented by formula (VIII): 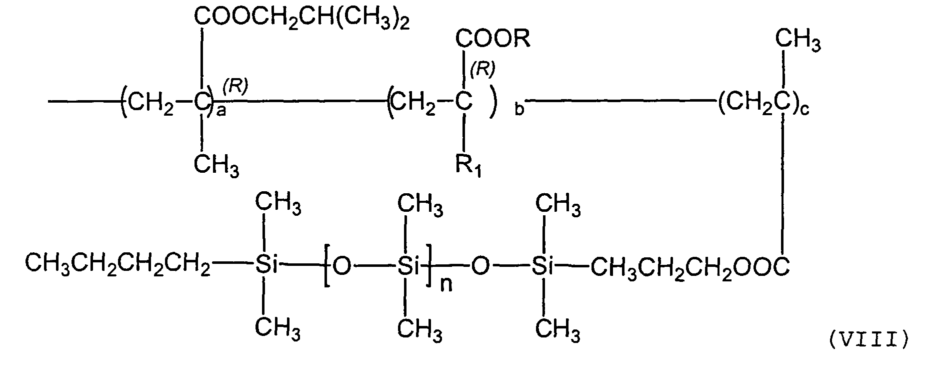 wherein

a, b, and c are present in a weight ratio of 69.9 : 0.1 : 30 respectively,

R and R¹, which may be identical or different, are each chosen from hydrogen and C₁-C₆ alkyl groups; and

m is a number ranging from 100 - 150. In an embodiment, m is chosen to provide a macromer having a molecular weight ranging from 8,000 to 12,000, such as 10,000. In another embodiment, m is a number ranging from 124-135, such as 130. Non-limiting examples of these copolymers are described in WO 01/32727 A1.

In another embodiment of the invention, the at least one copolymer comprises a backbone chosen from vinyl backbones, methacrylic backbones, and acrylic polymeric backbones and further comprises at least one pendant siloxane group. Non-limiting examples of such polymers are disclosed in U.S. Patents 4,693,935, 4,981,903 and 4,981,902.

In an embodiment, the at least one copolymer comprises at least one A monomer, at least one C monomer, and, optionally at least one B monomer, wherein the at least one A monomer is chosen from free-radically-polymerizable vinyl monomers, free-radically-polymerizable methacrylate monomers, and free-radically-polymerizable acrylate monomers; the at least one B monomer, if present, is chosen from at least one reinforcing monomer copolymerizable with the at least one A monomer, and the at least one C monomer is chosen from monomers represented by formula (IX):

X (Y)ₙ Si(R)₃₋ₘ Zₘ (IX)

wherein:

X is chosen from vinyl groups which are copolymerizable with the at least one A monomer and with the at least one B monomer;

Y is chosen from divalent groups;

n is zero or 1;

m is a number ranging from 1 to 3;

R, which may be identical or different, are each chosen from hydrogen, optionally substituted C₁-C₁₀ alkyl groups, optionally substituted phenyl groups, and optionally substituted C₁-C₁₀ alkoxy groups; and

Z, which may be identical or different, are each chosen from monovalent siloxane polymeric groups. Non-limiting examples of A monomers include methacrylic acid esters of C₁-C₁₂ linear alcohols, methacrylic acid esters of C₁₋C₁₂ of branched alcohols, styrene monomers, vinyl esters, vinyl chloride monomers, vinylidene chloride monomers, and acryloyl monomers. Non-limiting examples of B monomers include acrylic monomers comprising at least one group chosen from hydroxyl, amino, and ionic groups, and methacrylic monomers comprising at least one group chosen from hydroxyl, amino, and ionic groups. Non-limiting examples of ionic groups include quaternary ammonium groups, carboxylate salts, and sulfonic acid salts. The C monomers are as above defined above in preceding paragraphs.

In another embodiment of the invention, the at least one co-polymer is chosen from vinyl-silicone graft copolymers having the following formula and vinyl-silicone block copolymers represented by formula (X): 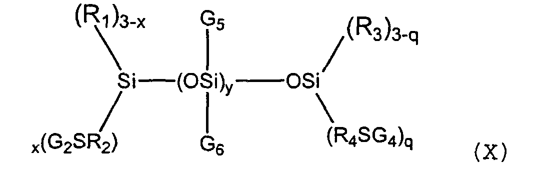 wherein

G₅, which may be identical or different, are each chosen from alkyl groups, aryl groups, aralkyl groups, alkoxy groups, alkylamino groups, fluoroalkyl groups, hydrogen, and - ZSA groups, wherein

A is chosen from vinyl polymeric segments comprising at least one polymerized free-radically-polymerizable monomer, and

Z is chosen from divalent C₁-C₁₀ alkylene groups, divalent aralkylene groups, divalent arylene groups, and divalent alkoxylalkylene groups. In an embodiment Z is chosen from methylene groups and propylene groups.

G₆, which may be identical or different, are each chosen from alkyl groups, aryl groups, aralkyl groups, alkoxy groups, alkylamino groups, fluoroalkyl groups, hydrogen, and - ZSA groups, as defined above;

G₂ comprises A;

G₄ comprises A;

R₁, which may be identical or different, are each chosen from alkyl groups, aryl groups, aralkyl groups, alkoxy groups, alkylamino groups, fluoroalkyl groups, hydrogen, and hydroxyl. In one embodiment, R₁ is chosen from C₁-C₄ alkyl groups, such as methyl groups, and hydroxyl.

R₂, which may be identical or different, are each chosen from divalent C₁₋₁₀ alkylene groups, divalent arylene groups, divalent aralkylene groups, and divalent alkoxyalkylene groups. In one embodiment, R₂ is chosen from divalent C₁-C₃ alkylene groups and divalent C₇-C₁₀ aralkylene groups. In another embodiment, R₂ is chosen from -CH₂ - groups and divalent 1,3-propylene groups.

R₃, which may be identical or different, are each chosen from alkyl groups, aryl groups, aralkyl groups alkoxy groups, alkylamino groups, fluoroalkyl groups, hydrogen, and hydroxyl. In one embodiment, R₃ is chosen from C₁-C₄ alkyl groups and hydroxyl. In another embodiment, R₃ is chosen from methyl groups.

R₄, which may be identical or different, are each chosen from divalent C₁-C₁₀ alkylene groups, divalent arylene groups, divalent aralkylene groups, and divalent alkoxyalkylene groups. In one embodiment, R₄ is chosen from divalent C₁-C₃ alkylene groups and divalent C₇-C₁₀ aralkylene groups. In another embodiment, R₄ is chosen from divalent -CH₂- groups and divalent 1,3-propylene groups.

x is a number ranging from 0 to 3;

y is a number greater than or equal to 5. In an embodiment, y ranges from 10 to 270, and in another embodiment, y ranges from 40 to 270.

q is a number ranging from 0 to 3;

Non-limiting examples of these polymers are described in U.S. Patent 5,468,477. A non-limiting example of such polymers is poly(dimethylsiloxane)-g-poly(isobutyl methacrylate), which is commercially available from 3M Company under the tradename VS 70 IBM.

In an embodiment, the at least one copolymer is present in the composition in an amount ranging from 0.1% to 20% by weight relative to the total weight of the composition. In another embodiment, the at least one copolymer is present in an amount ranging from 1% to 10% by weight relative to the total weight of the composition. One of ordinary skill in the art will recognize that the at least one copolymer according to the present invention may be commercially available, and may come from suppliers in the form of a dilute solution. The amounts of the at least one ccopolymer disclosed herein therefore reflect the weight percent of active material.

Other film forming polymers may also be a other non-silicone film formers. These non-silicone film formers may be chosen from, for example, polyethylene; vinylpyrrolidone/vinyl acetate (PVP/VA) copolymers such as the Luviskol® VA grades (all ranges) from BASF® Corporation and the PVP/VA series from ISP; acrylic fluorinated emulsion film formers including Foraperle® film formers such as Foraperle® 303 D from Elf Atochem (although Foraperle® may not be appropriate for some cosmetic formulations); GANEX® copolymers such as butylated PVP, PVP/Hexadecene copolymer, PVP/Eicosene copolymer or tricontanyl; Poly(vinylpyrrolidone/diethylaminoethyl methacrylate) or PVP/Dimethylaminoethylmethacrylate copolymers such as Copolymer 845; Resin ACO-5014 (Imidized 1B/MA copolymer); other PVP based polymers and copolymers; alkyl cycloalkylacrylate copolymers (*See* WO 98/42298, the disclosure of which is hereby incorporated by reference); Mexomere® film formers and other allyl stearate/vinyl acetate copolymers (allyl stearate/VA copolymers); polyolprepolymers such as PPG-12/SMDI copolymer, polyolprepolymers such as PPG-1 2/SM Dl copolymer, Poly(oxy-1,2-ethanediyl), α-hydro-ω-hydroxy-polymer with 1,1'-methylene-bis-(4- isocyanatocyclohexane) available from Barnet; Avalure™ AC Polymers (Acrylates Copolymer) and Avalure™ UR polymers (Polyurethane Dispersions), available from BF Goodrich.

The film former which also may be used within the framework of the invention includes film formers having any film former chemistry known in the art such as: PVP, acrylates, and urethanes; synthetic polymers of the polycondensate type or free-radical type, or ionic type, polymers of natural origin and mixtures thereof or any other film former known within the practice of the cosmetic and pharmaceutical arts which one skilled in the art may determine to be compatible. Film formers that may be used are also disclosed in the *International Cosmetic Ingredient Dictionary and Handbook Vol. 2* (7^{th} ed. 1999) , more particularly the emollients disclosed on pages 1636-1638.

The compositions of the present invention may further include one or more structuring agents e.g., to increase viscosity or otherwise modify the rheology of said compositions. Suitable structuring agents will generally be semi-solid or solid at room temperature and include waxes or wax-like materials such as fatty alcohols, fatty acids, and natural, synthetic, or hydrocarbon waxes. In some embodiments, structuring agents have a melting point of 30 to 120 °C, and can be animal waxes, plant waxes, mineral waxes, silicone waxes, synthetic waxes, and petroleum waxes or fatty acids or fatty alcohols. More specific examples of waxes include bayberry, beeswax, candelilla, carnauba, ceresin, cetyl esters, hydrogenated jojoba oil, hydrogenated jojoba wax, hydrogenated microcrystalline wax, hydrogenated rice bran wax, japan wax, jojoba butter, jojoba esters, jojoba wax, lanolin wax, microcrystalline wax, mink wax, montan acid wax, montan wax, ouricury wax, ozokerite, paraffin, PEG-6 beeswax, PEG-8 beeswax, rice bran wax, shellac wax, spent grain wax, sulfurized jojoba oil, synthetic beeswax, synthetic candelilla wax, synthetic carnauba wax, synthetic japan wax, synthetic jojoba oil, ethylene homo- or copolymers, stearoxy dimethicone, dimethicone behenate, shorea stenoptera butter, stearyl dimethicone, and the like, as well synthetic homo- and copolymer waxes such as PVP/eicosene copolymer, PVP/hexadecene copolymer, and the like.

Also suitable as structuring agents are one or more fatty alcohols having the formula RCH₂OH wherein R is a straight or branched chain saturated or unsaturated alkyl having at least about 6 to 30 carbon atoms. Examples of fatty alcohols suitable for use include behenyl alcohol, C₉₋₁₅ alcohols, caprylic alcohol, cetearyl alcohol, cetyl alcohol, coconut alcohol, decyl alcohol, lauryl alcohol, cetyl alcohol, myristyl alcohol, oleyl alcohol, palm alcohol, stearyl alcohol, tallow alcohol, and the like. The preferred compositions of the invention include a mixture of cetyl and stearyl alcohols. Structuring agents are also disclosed in patent publications US20020076386 and US2002018159.

The compositions of the present invention may contain a "deodorant active agent". Such agents (1) reduce the flow of sweat and (2) mask, (3) improve and/or (4) reduce an unpleasant odor resulting from the bacterial decomposition of human sweat. Deodorant active agents include antiperspirant compounds, alum salts, bacteriostatic agents, bactericidal agents (such as, for example, 2,4,4'-trichloro-2'-hydroxydiphenyl ether and 3,7,11-trimethyldodeca-2,5,10-trienol), various zinc salts and odor-absorbing agents (such as sodium bicarbonate and zinc pidolate (zinc pyrrolidonecarboxylate)). Non-limiting examples of antiperspirant compounds include aluminum salts (such as, for example, aluminum chlorohydrate, aluminum hydroxychloride), zirconium salts and aluminum and zirconium salts. 3,7,11-Trimethyldodeca-2,5,10-trienol is, for example, sold under the name Farnesol™ by Dragoco and 2,4,4'-trichloro-2'-hydroxydiphenyl ether is sold under the name Irgacare™ MP by Ciba-Geigy. Non-limiting examples of aluminum salts that may be used as the at least one deodorant active agent according to the present invention include aluminum hydroxychloride, sold by Reheis under the trade name Reach 301 and by Guilini Chemie under the trade name Aloxicoll PF 40. Non-limiting examples of aluminum and zirconium salts that may be used according to the present invention include the salt sold by the company Reheis under the trade name Reach A2P-908-SUF. Complexes of zirconium hydroxychloride and aluminum hydroxychloride and glycine, commonly known under the name "ZAG complexes," may also be used according to the invention.

The cosmetic compositions of this invention may also comprise sunscreens which are chemical absorbers actually absorb harmful ultraviolet radiation. It is well known that chemical absorbers are classified, depending on the type of radiation they protect against, as either UV-A or W-B absorbers. UV-A absorbers generally absorb radiation in the 320 to 400 nm region of the ultraviolet spectrum. UV-A absorbers include anthranilates, benzophenones, and dibenzoyl methanes. UV-B absorbers generally absorb radiation in the 280 to 320 nm region of the ultraviolet spectrum. UV-B absorbers include p-aminobenzoic acid derivatives, camphor derivatives, cinnamates, and salicylates.

Classifying the chemical absorbers generally as UV-A or UV-B absorbers is accepted within the industry. However, a more precise classification is one based upon the chemical properties of the sunscreens. There are eight major classifications of sunscreen chemical properties which are discussed at length in "Sunscreens - Development, Evaluation and Regulatory Aspects," by N. Shaath et al., 2nd. Edition, pages 269-273, Marcel Dekker, Inc. (1997).

The sunscreens useful in the present invention typically comprise chemical absorbers, but may also comprise physical blockers. Exemplary sunscreens which may be formulated into the compositions of the present invention are chemical absorbers such as p-aminobenzoic acid derivatives, anthranilates, benzophenones, camphor derivatives, cinnamic derivatives, dibenzoyl methanes (such as avobenzone also known as Parsol®1789), diphenylacrylate derivatives, salicylic derivatives, triazine derivatives, benzimidazole compounds, bis-benzoazolyl derivatives, methylene bis-(hydroxyphenylbenzotriazole) compounds, the sunscreen polymers and silicones, or mixtures thereof. These are variously described in U.S. Patents 2,463,264, 4,367,390, 5,166,355 and 5,237,071 and in EP 863,145, EP 517,104, EP 570,838, EP 796,851, EP 775,698, EP 878,469, EP 933,376, EP 893,119, EP 669,323, GB 2,303,549, DE 1,972,184 and WO 93/04665. Also exemplary of the sunscreens which may be formulated into the compositions of this invention are physical blockers such as cerium oxides, chromium oxides, cobalt oxides, iron oxides, red petrolatum, silicone-treated titanium dioxide, titanium dioxide, zinc oxide, and/or zirconium oxide, or mixtures thereof.

A wide variety of sunscreens is described in U.S. Patents 5,087,445 and 5,073,372, and Chapter VIII of Cosmetics and Science and Technology (1957) by Segarin *et al*., pages 189 et seq.

Sunscreens which may be formulated into the compositions of the instant invention are those selected from among: aminobenzoic acid, amyldimethyl PABA, cinoxate, diethanolamine *p*-methoxycinnamate, digalloyl trioleate, dioxybenzone, 2-ethoxyethyl p-methoxycinnamate, ethyl 4-bis(hydroxypropyl)aminobenzoate, 2-ethylhexyl-2-cyano-3,3-diphenylacrylate, ethylhexyl *p*-methoxycinnamate, 2-ethylhexyl salicylate, glyceryl aminobenzoate, homomenthyl salicylate, homosalate, 3-imidazol-4-ylacrylic acid and ethyl ester, methyl anthranilate, octyldimethyl PABA, 2-phenylbenzimidazole-5-sulfonic acid and salts, red petrolatum, sulisobenzone, titanium dioxide, triethanolamine salicylate, *N*, *N*, *N*-trimethyl-4-(2-oxoborn-3-ylidene methyl)anillinium methyl sulfate, and mixtures thereof.

Sunscreens active in the UV-A and/or UV-B range can also include:
p-aminobenzoic acid,
oxyethylene (25 mol) p-aminobenzoate,
2-ethylhexyl p-dimethylaminobenzoate,
ethyl N-oxypropylene p-aminobenzoate,
glycerol p-aminobenzoate,
4-isopropylbenzyl salicylate,
2-ethylhexyl 4-methoxycinnamate,
methyl diisopropylcinnamate,
isoamyl 4-methoxycinnamate,
diethanolamine 4-methoxycinnamate,
3-(4'-trimethylammunium)-benzyliden-bornan-2-one methylsulfate,
2-hydroxy-4-methoxybenzophenone,
2-hydroxy-4-methoxybenzophenone-5-sulfonate,
2,4-dihydroxybenzophenone,
2,2',4,4'-tetrahydroxybenzophenone,
2,2'-dihydroxy-4,4'dimethoxybenzophenone,
2-hydroxy-4-n-octoxybenzophenone,
2-hydroxy-4-methoxy-4'-methoxybenzophenone,
-(2-oxoborn-3-ylidene)-tolyl-4-sulfonic acid and soluble salts thereof,
3-(4'-sulfo)benzyliden-bornan-2-one and soluble salts thereof,
3-(4'methylbenzylidene)-d,1-camphor,
3-benzylidene-d,1-camphor,
benzene 1,4-di(3-methylidene-10-camphosulfonic) acid and salts thereof (the product Mexoryl SX described in U.S. Patent 4,585,597,
urocanic acid,
2,4,6-tris[p-(2'-ethylhexyl-1'-oxycarbonyl)-anilino]-1,3,5-triazine,
2-[(p-(tertiobutylamido)anilino]-4,6-bis- [(p-(2'-ethylhexyl-1'-oxycarbonyl)anilino]-1,3,5-triazine,
2,4-bis{[4-(2-ethyl-hexyloxy)]-2-hydroxy]-phenyl}-6-(4-methoxy-phenyl)-1,3,5-triazine ("TINOSORB S" marketed by Ciba) ,
the polymer of N- (2 et 4) - [(2-oxoborn-3-yliden)methyl]benzyl]-acrylamide,
1,4-bisbenzimidazolyl-phenylen-3,3',5,5'-tetrasulfonic acid and salts thereof,
the benzalmalonate-substituted polyorganosiloxanes,
the benzotriazole-substituted polyorganosiloxanes (Drometrizole Trisiloxane),
dispersed 2,2'-methylene-bis-[6-(2H-benzotriazol-2-yl)-4-(1,1,3,3-tetramethylbutyl)phenol] such as that marketed under the trademark MIXXIM BB/100 by Fairmount Chemical, or micronized in dispersed form thereof such as that were marketed under the trademark TINOSORB M by Ciba Specialty Chemicals Corp. (Tarrytown, NY), and
solubilized 2,2'-methylene-bis-[6-(2H-benzotriazol-2-yl)-4-(methyl)phenol] such as that marketed under the trademark MIXXIM BB/200 by Fairmount Chemical.

Typically, combinations of one of more of these sunscreens are used.

The dibenzoyl methane derivatives other than avobenzone are described, for example, in FR 2,326,405, FR 2,440,933 and EP 114,607.

Other dibenzoyl methane sunscreens other than avobenzone include (whether singly or in any combination):
2-methyldibenzoylmethane;
4-methyldibenzoylmethane;
4-isopropyldibenzoylmethane;
4-tert-butyldibenzoylmethane;
2,4-dimethyldibenzoylmethane;
2,5-dimethyldibenzoylmethane;
4,4'-diisopropyldibenzoylmethane;
4,4'-dimethoxydibenzoylmethane;
2-methyl-5-isopropyl-4'-methoxydibenzoylmethane;
2-methyl-5-tert-butyl-4'-methoxydibenzoylmethane;
2,4-dimethyl-4'-methoxydibenzoylmethane; and
2,6-dimethyl-4-tert-butyl-4'-methoxydibenzoylmethane.

Additional sunscreens that can be used are described in pages 2954-2955 of the International *Cosmetic Ingredient Dictionary and Handbook* (9^{th} ed. 2002).

Plasticizers may also be added to the compositions to improve the flexibility and cosmetic properties of the resulting formulation. Plasticizers are materials that soften synthetic polymers. They are frequently required to avoid brittleness and cracking of film formers. One skilled in the art may routinely vary the amount of plasticizer desired based on the properties desired and the application envisaged. Plasticizers useful in the practice of the invention include lecithin, polysorbates, dimethicone copolyol, glycols, citrate esters, glycerin, dimethicone, and other similar ingredients disclosed in the *International Cosmetic Ingredient Dictionary and Handbook Vol. 4* (9^{th} ed. 2002), more particularly the plasticizers disclosed on page 2927.

The composition of the present invention may also further comprise at least one suitable (e.g., cosmetically or dermatologically acceptable) additive commonly used in the field concerned chosen from coloring agents (e.g., pigments), antioxidants, essential oils, preserving agents, fragrances, fillers, pasty fatty substances, waxy fatty substances, neutralizing agents, lipo-soluble polymers, and cosmetically active agents and dermatological active agents such as, for example, emollients, moisturizers, vitamins and essential fatty acids. The compositions of the invention may also be optionally thickened with an aqueous-phase thickener or gelled with a gelling agent and/or containing ingredients soluble in water. In embodiments where the cosmetic compositions are colored due to the presence of at least one pigment, the pigment is preferably treated, e.g., with an amino acid. Treated pigments are known in the art. See, e.g., U.S. Patent 5,843,417. For example, pigments treated with silicones are described in U.S. Patent 4,574,082, and pigments treated with amino acids are described in U.S. Patent 4,606,914. Treated pigments are commercially available from U.S. Cosmetics Corp., a distributor of Miyoshi Kasei (Japan) (e.g., pigments treated with a vegetable-derived amino acid such as disodium stearoyl glutamate, aluminum oxide and optionally titanium dioxide).

The invention will be further described by reference to the detailed examples. These examples are provided for purposes of illustration only, and are not intended to be limiting unless otherwise specified.

### Example 1: Emulsion Foundation Formula

| Phase | Trade Name | INCI Name | %w/w |
|---|---|---|---|
| | | | |
| A1 | Versagel MD 500 | Isododecane (and) Ethylene/Propylene/Styrene Copolymer (and) Butylene/ethylene/Styrene Copolymer | 15.00 |
| | Hostacerin DGI | Polyglyceryl-2 sesquiisostearate | 2.00 |
| | PERMETHYL 99A | Isododecane | 13.20 |
| | Wickenol 151 | Isononyl Isononanoate | 4.00 |
| | ITT-Titanium Dioxide | ITT-Titanium Dioxide | 10.34 |
| | ITT-Iron Oxide - Yellow | ITT-Iron Oxides | 1.10 |
| | ITT-Iron Oxide - Red | ITT-Iron Oxides (and) Iron Oxides | 0.36 |
| | ITT-Iron Oxide - Black | ITT-Iron Oxides (and) Iron Oxides | 0.20 |
| | | | |
| A2 | CARDRE MICA 8 | Mica | 3.00 |
| | MSS-500W | SILICA | 4.00 |
| | DC 200 Fluid 10 cst | Dimethicone | 6.00 |
| | KSP-100 | VINYL DIMETHICONE/METHICONE SILSESQUIOXANE CROSSPOLYMER | 3.00 |
| | | | |
| B | Water | Water | 35.00 |
| | SODIUM CHLORIDE | SODIUM CHLORIDE | 1.00 |
| | HYDROLITE-5 | PENTYLENE GLYCOL | 0.50 |
| | SODIUM DEHYDROACETATE MONOHYDRATE | SODIUM DEHYDROACETATE | 0.20 |
| | Methylparaben | Methylparaben | 0.20 |
| | BRIJ 30 | Laureth-4 | 0.50 |
| | Phenoxyethanol | Phenoxyethanol | 0.40 |
| | | | |
| | | Total: | 100.00 |

The emulsion was prepared by mixing phase A ingredients, grinding pigments with Silverson at 5500 to 7000 rpm at room temperature for 20 to 30 min, or until good dispersion, and adding phase A2 ingredients one at a time and dispersing well. In a separate beaker, phase B ingredients were heated to 55-60 °C while stirring, and then cooled to room temperature, followed by adding to Phase A slowly to emulsion with homogenization.

### Example 2: Anhydrous Make-up Foundation

| Phase | Trade Name | INCI Name | %w/w |
|---|---|---|---|
| | | | |
| A | INX70UB | Titanium dioxide (and) ISONONYL ISONONANOATE (and) ISOPROPYL TITANIUM TRIISOSTEARATE | 15.54 |
| | INX70ER | IRON OXIDES (and) ISONONYL ISONONANOATE (and) ISOPROPYL TITANIUM TRIISOSTEARATE | 3.2 |
| | INX55EY | IRON OXIDES (and) ISONONYL ISONONANOATE (and) ISOPROPYL TITANIUM TRIISOSTEARATE | 1.50 |
| | INX70EB | IRON OXIDES (and) ISONONYL ISONONANOATE (and) ISOPROPYL TITANIUM TRIISOSTEARATE | 0.96 |
| | PERMETHYL 99A | Isododecane | 22.00 |
| | | | |
| B | Rhapsody 3R | Talc | 6.00 |
| | MSS-500W | SILICA | 23.00 |
| | DC 200 Fluid 10 cst | Dimethicone | 3.70 |
| | KSP-100 | VINYL DIMETHICONE/METHICONE SILSESQUIOXANE CROSSPOLYMER | 1.60 |
| | | | |
| C | Versagel MD 500 | Isododecane (and) Ethylene/Propylene/Styrene Copolymer (and) Butylene/ethylene/Styrene Copolymer | 18.00 |
| | Versagel MD 1600 | Isododecane (and) Ethylene/Propylene/Styrene Copolymer (and) Butylene/ethylene/Styrene Copolymer | 4.50 |
| | | | |
| | | Total: | 100.00 |

To prepare the foundation, mix phase A ingredients at room temperature, until uniform;

add phase B ingredients one at a time with stirring until good dispersion, and then add Phase C ingredients and mix to uniformity.

All publications cited in the specification, both patent publications and non-patent publications, are indicative of the level of skill of those skilled in the art to which this invention pertains. All these publications are herein incorporated by reference to the same extent as if each individual publication were specifically and individually indicated as being incorporated by reference.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A cosmetic composition, comprising a gelled block copolymer having at least one hard segment and at least one soft segment; and a silicone elastomer powder comprising a silicone elastomer core coated with a silicone resin, wherein said powder is swelled with a swelling agent.

2. A composition according to claim 1, wherein the hard segment comprises polymethacrylate, polyacrylate or polystyrene.

3. A composition according to claim 2, wherein the hard segment comprises polystyrene.

4. A composition according to claim 1, 2 or 3, wherein the soft segment comprises an ethylene/propylene copolymer, an ethylene/butylene copolymer, polybutylene, polyisoprene, an hydrogenated butadienene polymer, an hydrogenated isoprene polymer, butadiene, or a mixture thereof.

5. A composition according to claim 4, wherein the soft segment comprises an ethylene/propylene copolymer or an ethylene/butylene copolymer.

6. A composition according to any one of the preceding claims wherein the block copolymer has a di-block configuration.

7. A composition according to claim 6, wherein said block copolymer comprises styrene-ethylenepropylene; styrene-ethylenebutylene; styrene-butadiene; or styrene-isoprene.

8. A composition according to any one of claims 1 to 5, wherein said block copolymer has a tri-block configuration.

9. A composition according to claim 8, wherein said block copolymer comprises styrene-butadiene-styrene; styrene-isoprene-styrene; styrene-ethylenebutylene-styrene; styrene-butylene-ethylene-styrene; or styrene-ethylenepropylene-styrene.

10. A composition according to any one of claims 1 to 5, wherein said block copolymer has a radial or star block configuration.

11. A composition according to claim 10, wherein said block copolymer comprises ethylene-propylene-styrene.

12. A composition according to any one of the preceding claims, wherein said at least one hard segment has a glass transition temperature of at least 60°C.

13. A composition according to any one of claims 1 to 11, wherein said at least one soft segment has a glass transition temperature no higher than room temperature.

14. A composition according to any one of the preceding claims, wherein said gelled block copolymer comprises an oil, hydrocarbon or an ester.

15. A composition according to claim 14 wherein the hydrocarbon comprises isododecane.

16. A composition according to any one of the preceding claims, wherein said gelled copolymer is present in an amount of about 0.1% to about 95% of said composition.

17. A composition according to any one of the preceding claims, wherein said at least one hard segment is present in an amount of about 10% to about 40% of total weight of said copolymer.

18. A cosmetic composition according to any one of the preceding claims, wherein said swelling agent comprises a linear or cyclic polydimethylsiloxane.

19. A cosmetic composition according to claim 18, wherein said polydimethylsiloxane comprises a cyclomethicone; or a dimethicone.

20. A cosmetic composition according to any one of claims 1 to 17, wherein said swelling agent comprises a phenylmethicone, a fluorinated silicone; or a polyorganosilsesquioxane.

21. A cosmetic composition according to any one of the preceding claims, wherein said silicone elastomer core is unfunctionalised.

22. A cosmetic composition according to any one of claims 1 to 20, wherein said silicone elastomer core contains pendant functional groups.

23. A cosmetic composition according to claim 22, wherein said functional groups comprise fluoroalkyl groups or phenyl groups.

24. A cosmetic composition according to any one of the preceding claims, further comprising a liquid fatty phase comprising a polar oil, an apolar oil, or a mixture of said polar and apolar oils; a hydrocarbon; or an ester.

25. A cosmetic composition according to any one of the preceding claims, further comprising an aqueous phase.

26. A cosmetic composition according to claim 25, further comprising an emulsifier, and which is in the form of an emulsion.

27. A cosmetic composition according to any one of claims 1 to 24, which is anhydrous.

28. A cosmetic composition according to any one of the preceding claims, further comprising a film-forming agent; a wax; a sunscreen agent; a plasticiser; an additive; and/or a pigment.

29. A cosmetic composition according to any one of the preceding claims, which is in the form of a solid, a paste, a gel or a cream.

30. A cosmetic composition according to any one of the preceding claims, which is in a moulded form.

31. A cosmetic composition according to any one of the preceding claims, which is in the form of a stick or dish.

32. A cosmetic composition according to any one of claims 1 to 30, which is in the form of a powder.

33. A cosmetic composition according to any one of the preceding claims, wherein the ratio of the amount of said silicone elastomer powder to said gelled block copolymer is from about 0.1 to about 9.0.

34. A method for care, make-up or treatment of a keratin material, comprising applying to the keratin material a composition comprising a gelled block copolymer having at least one hard segment and at least one soft segment; and a silicone elastomer powder comprising a silicone elastomer core coated with a silicone resin, wherein said powder is swelled with a swelling agent.

35. A method according to claim 34, wherein the keratin material comprises lips; skin; or keratinous fibres.
